# DEMANDE DE BREVET EUROPEEN

(11) **EP 0 872 224 A1**
(43) Date de publication de la demande: **21.10.1998**
(21) Numéro de dépôt: 98490012.6
(22) Date de dépôt: 16.04.1998
(51) Int. Cl.: A61F 5/01, A61F 2/68

(54) **Dispositif d'articulation pour prothèse des membres inférieurs et notamment du genou**

(30) Priorité: 17.04.1997 FR 9704984
(71) Demandeur: Nord Orthopedie S.A., 59120 Loos (FR)
(72) Inventeur: Barbez, Philippe, 59223 Roncq (FR)
(74) Mandataire: Ecrepont, Robert

(57) **Abrégé**

L'invention se rapporte à un dispositif d'articulation notamment de genou comprenant une première partie (2), une deuxième partie (3), une articulation (4) reliant les deux parties précitées, un moyen (5) de verrouillage en rotation de l'articulation et un détecteur de poids.

Ce dispositif d'articulation est caractérisé en ce que le détecteur de charge consiste en une poche contenant un fluide qui, au travers d'une conduite, actionne le moyen de verrouillage.

## Description

L'invention se rapporte à un dispositif d'articulation pour orthèse des membres inférieurs et notamment du genou.

L'orthèse est un système mécanique suppléant un membre déficient ou paralysé qui ne permet plus de supporter la charge qu'il doit notamment supporter, tel pour la jambe le poids du corps.

Pour comprendre le besoin d'utiliser une orthèse du genou, il faut avant tout analyser la marche.

Le cycle de la marche peut se décomposer en quatre phases dont on considère généralement que le point de départ est l'instant où le poids du corps est réparti à 50% sur chaque membre.

Pour une orthèse du genou droit, on considéra alors que le point de départ commence lorsque le membre droit est en arrière.

Dans cette première phase, la pression s'exerçant sur le genou droit diminue et est reportée progressivement sur le genou gauche valide.

Lorsque le poids du corps repose sur la jambe gauche, la jambe droite est ramenée vers l'avant et la flexion jambe/cuisse augmente.

La troisième phase commence lorsque le talon du pied droit repose sur le sol et qu'une partie du poids du corps se reporte sur ce genou droit.

Lors de la quatrième phase, le poids s'exerçant sur la jambe droite augmente progressivement et la jambe gauche peut alors être ramenée vers l'avant.

C'est principalement lors de la troisième phase et de la quatrième phase que l'orthèse doit suppléer à la défaillance du genou et c'est pendant la troisième phase que les conditions sont extrêmes.

Classiquement, un dispositif d'articulation, notamment du genou, comprend :
- une première partie ajustée sur la cuisse,
- une deuxième partie ajustée sur la jambe et
- une articulation reliant les deux parties précitées.

Pour éviter, lors de la marche, que le membre inférieur se dérobe sous le poids de l'individu en raison de la déficience du genou, il est connu de prévoir sur l'articulation de l'orthèse un verrouillage en rotation.

Ainsi, l'articulation est verrouillée en rotation lorsque le membre inférieur est tendu et, pour permettre la flexion, on déverrouillera momentanément la dite articulation.

Cette méthode a pour inconvénient de condamner la flexion du genou pour les patients dont les défaillances ne sont qu'occasionnelles.

Par ailleurs, le déverrouillage nécessite une action volontaire sur un moyen de commande.

Au lieu d'un verrouillage en rotation, il est connu WO-A-95.06499 d'équiper ces articulations d'un frein.

L'action du frein peut être variable suivant l'orientation que la jambe prend par rapport à la cuisse au cours de la marche, mais les réglages sont délicats et doivent être souvent refaits en raison de l'usure.

On connaît une orthèse (US-2.943.622) présentant un dispositif de verrouillage actionné par le talon du porteur.

Pour cela, la chaussure est équipée d'une palette articulée sur un axe.

Sous l'effet du porteur, la palette bascule et pousse sur un câble voire une tige rigide qui commande un loquet muni de plusieurs dents qui s'engrènent avec un secteur denté d'une roue dentée calée en rotation sur la partie haute de l'orthèse.

Ce dispositif présente divers inconvénients :
- le premier réside dans le flambage de la tige entraînant rapidement un disfonctionnement de l'orthèse,
- le second inconvénient est lié à la présence de la commande dans la chaussure qui gêne la marche.

Un des résultats que l'invention vise à obtenir est une orthèse du type cité plus haut qui remédie à ces inconvénients.

L'invention a pour objet un dispositif d'articulation pour membre inférieur comprenant :
- une première partie notamment ajustée sur la cuisse,
- une deuxième partie notamment ajustée sur la jambe,
- une articulation reliant les deux parties précitées et
- un moyen de verrouillage en rotation de l'articulation, ce dispositif d'articulation étant caractérisé en ce que

ce dispositif étant caractérisé en ce que le détecteur de charge consiste en une poche contenant un fluide qui, au travers d'une conduite, actionne le moyen de verrouillage.

L'invention sera bien comprise à l'aide de la description ci-après faites à titre d'exemple non limitatif en regard du dessin ci-annexé qui représente schématiquement :
- figure 1 : un dispositif d'articulation selon l'invention,
- figure 2 : un détail du dispositif d'articulation en perspective,
- figure 3 : le détail de la figure 2 vue selon une autre direction,
- figure 4 : un détail de la figure 2 ou 3,
- figure 5 : une vue en gros plan d'un dispositif de verrouillage.

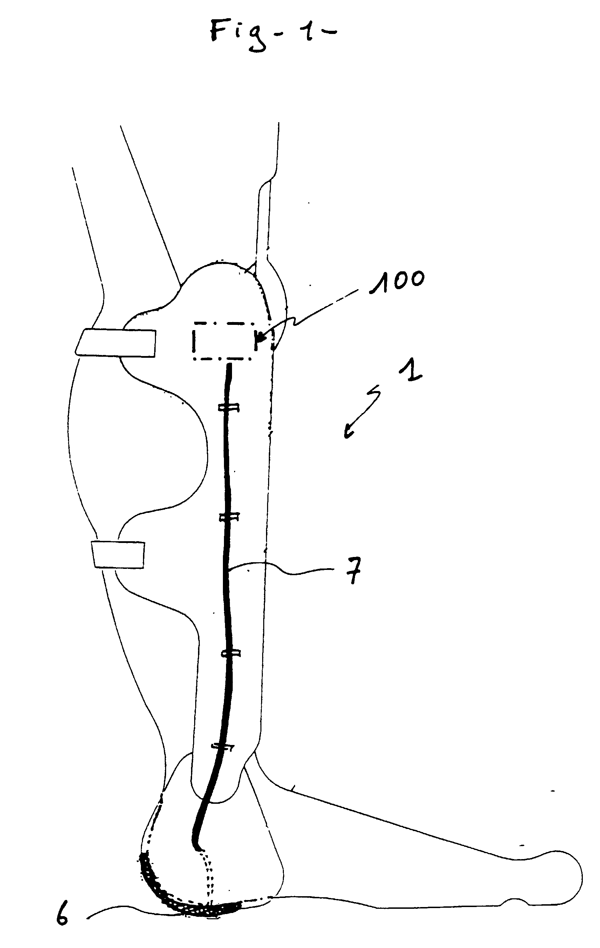

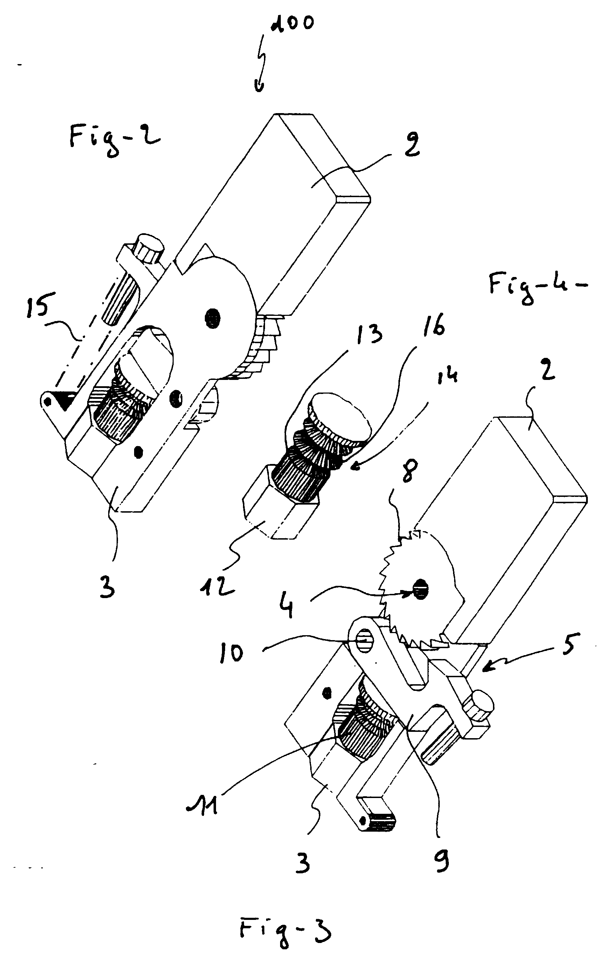

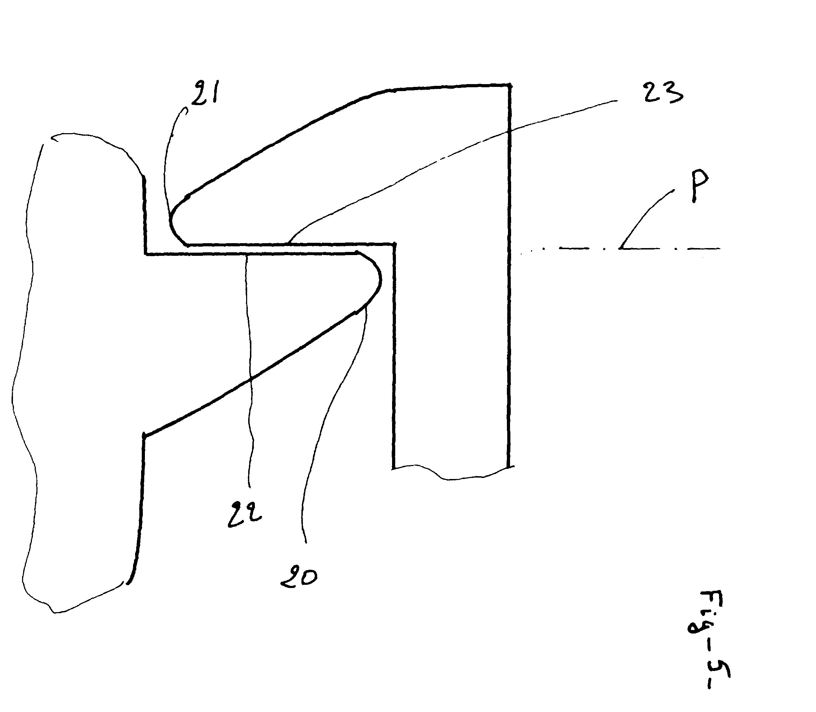

En se reportant au dessin, on voit que l'orthèse 1 de membre inférieur pour pallier à la déficience du genou comprend :
- une première partie 2 notamment ajustée sur la cuisse,
- une deuxième partie 3 notamment ajustée sur la jambe,
- une articulation 4 reliant les deux parties précitées et
- un moyen 5 de verrouillage en rotation de l'articulation.

Ce dispositif d'articulation permet la marche et autorise la flexion du membre inférieur.

Le dispositif d'articulation comprend, sous le pied du membre inférieur considéré, un détecteur 6 de poids qui, au delà d'une valeur de charge prédéterminée, commande le verrouillage en rotation de l'articulation 4.

Selon l'invention, le détecteur 6 de charge consiste en une poche 6 contenant un fluide qui, au travers d'une conduite 7, actionne le moyen de verrouillage.

Le fluide peut être faiblement compressible, tel un liquide.

La compressibilité du fluide peut être ajusté en introduisant de l'air ou un autre gaz avec le fluide.

Cela permet d'agir sur la vitesse de verrouillage.

Le moyen 5 de verrouillage en rotation interdit la flexion du membre inférieur mais autorise cependant son extension lorsque ce dispositif de verrouillage est activé.

Egalement, ce moyen de verrouillage autorise une amplitude de rotation de quelques degrés d'une partie par rapport à l'autre même pendant la phase de verrouillage.

Ce moyen 5 de verrouillage comprend un secteur denté 8 et un loquet 9 articulé autour d'un axe 10 transversal à la marche et déplacé d'une position neutre vers sa position de verrouillage par un actionneur 11 relié au détecteur 6 de charge.

Avantageusement, le moyen de verrouillage est une roue à rochet, c'est à dire une roue à dents taillées en biseau de façon à ne pouvoir soulever que, dans un sens, un cliquet qui l'immobilise en rotation dans l'autre sens.

Les dents de la roue à rochet et la dent que présente le cliquet ont leurs extrémités libres 20, 21 arrondies de sorte que cela évite d'abîmer les dents lors du verrouillage si celui-ci est trop tardif.

La face 22 d'appui de la dent portée par la roue dentée et celle 23 du cliquet sont dans un plan radial à la roue ou incliné par rapport à ce plan.

Avantageusement, l'actionneur 11 se présente sous la forme extérieure d'une ventouse comprenant une base 12 de laquelle s'élève une paroi 13 cylindrique comprenant des moyens 14 pour que sa dimension axiale soit variable.

Cette ventouse est reliée à la conduite 7 elle-même reliée à la poche précitée.

Cet actionneur simple effet est ramené en sa position neutre par un moyen de rappel 16 constitué notamment par l'élasticité de sa paroi latérale en forme de soufflet.

En complément de cette élasticité, est prévu un ressort 15 de rappel.

L'actionneur sera logé dans une cavité que présente l'une des deux pièces mobiles de l'articulation.

Cet actionneur sera guidé en translation par exemple à l'aide d'un manchon.

La taille de la poche est déterminée en fonction des divers paramètres que sont :
- la force à appliquer sur le cliquet pour éviter un déverrouillage accidentel, la hauteur de l'articulation par rapport au sol et la perte de charge engendrée par la conduite allant de la poche à l'actionneur,
- la compressibilité du fluide et
- le poids minimal permettant le verrouillage.

Eventuellement, on peut prévoir un moyen moteur tel un vérin facilitant le passage en extension du membre.

## Revendications

1. Dispositif d'articulation pour orthèse des membres inférieurs et notamment de genou comprenant :
- une première partie (2),
- une deuxième partie (3),
- une articulation (4) reliant les deux parties précitées,
- un moyen (5) de verrouillage en rotation de l'articulation, et
- un détecteur (6) de poids qui commande le verrouillage en rotation de l'articulation (4),
ce dispositif étant **CARACTERISE** en ce le détecteur (6) de charge consiste en une poche (6) contenant un fluide qui, au travers d'une conduite (7), actionne le moyen de verrouillage.

2. Dispositif d'articulation selon la revendication 1 **caractérisé** en ce que le fluide est faiblement compressible.

3. Dispositif d'articulation selon la revendication 1 **caractérisé** en ce que la compressibilité du fluide est ajustée en introduisant de l'air ou un autre gaz avec le fluide.

4. Dispositif d'articulation selon la revendication 1 **caractérisé** en ce que le moyen (5) de verrouillage en rotation est un moyen pour interdire la flexion du membre inférieur mais autoriser cependant son extension.

5. Dispositif d'articulation selon la revendication 1 **caractérisé** en ce que le moyen de verrouillage autorise une amplitude de rotation de quelques degrés d'une partie par rapport à l'autre pendant la phase de verrouillage.

6. Dispositif d'articulation selon la revendication 4 ou 5 **caractérisé** en ce que le moyen (5) de verrouillage est une roue à rochet.

7. Dispositif d'articulation selon la revendication 1 ou 6 **caractérisé** en ce que les dents de la roue à rochet et la dent que présente le cliquet ont leurs extrémités libres (20, 21) arrondies.

8. Dispositif d'articulation selon la revendication 7 **caractérisé** en ce que le cliquet est déplacé vers sa position de verrouillage par un actionneur (11) se présentant sous la forme extérieure d'une ventouse comprenant une base (12) de laquelle s'élève une paroi (13) cylindrique comprenant des moyens (14) pour que sa dimension axiale soit variable.

9. Dispositif d'articulation selon la revendication 7 ou 8 **caractérisé** en ce que l'actionneur (11) simple effet est ramené en sa position neutre par un moyen de rappel (16) constitué notamment par l'élasticité de sa paroi latérale en forme de soufflet.

10. Dispositif d'articulation selon la revendication 9 **caractérisé** en ce qu'il comprend un ressort (15) de rappel.
